Europäisches Patentamt

European Patent Office

Office européen des brevets

(19)

(11) Publication number : **0 633 321 A1**

# (12) EUROPEAN PATENT APPLICATION

(21) Application number : **94304237.4**

(22) Date of filing : **13.06.94**

(51) Int. Cl.[6] : **C12Q 1/70,** C12Q 1/68, C07H 21/04, C12P 19/34

(30) Priority : **14.06.93 JP 179753/93**

(43) Date of publication of application :
**11.01.95 Bulletin 95/02**

(84) Designated Contracting States :
**BE CH DE DK FR GB IT LI NL**

(71) Applicant : **IMMUNO JAPAN INC.**
**4-28-14-701, Ogikubo**
**Suginami-Ku**
**Tokyo 167 (JP)**

(72) Inventor : **Okamoto, Hiroaki**
**1560-25, Ishibashi,**
**Ishibashi-Machi**
**Shimotsuga-gun, Tochigi 329-05 (JP)**

(74) Representative : **Arthur, Bryan Edward**
**Withers & Rogers**
**4 Dyers Buildings**
**Holborn**
**London, EC1N 2JT (GB)**

(54) **HCV related oligonucleotides and uses thereof in methods for detecting and genotyping of HCV, and kits used in these methods.**

(57)     One method of determining the HCV genotype of a HCV strain is provided which involves subjecting the HCV strain to two stage PCR, wherein the first stage PCR involves utilizing antisense primer #186 (SEQ ID NO :5) and at least one of sense primers #256 (SEQ ID NO :4) and #256V (SEQ ID NO :1) for first stage amplification and wherein the second stage PCR involves utilizing at least one of sense primers #104 (SEQ ID NO :6) and #104V (SEQ ID NO :2) and at least one of antisense primers #296 (SEQ ID NO :7), #133 (SEQ ID NO :8), #134 (SEQ ID NO :9), #135 (SEQ ID NO :10), and #339 (SEQ ID NO :3) for the second stage amplification ; the method further involves comparing the product of the second stage PCR with HCV genotypes I, II, III, IV, and V. The method may further involve synthesizing cDNA from viral RNA of the HCV strain and/or measuring the molecular weight of the product of the second stage PCR.

EP 0 633 321 A1

Jouve, 18, rue Saint-Denis, 75001 PARIS

## REFERENCE TO A RELATED APPLICATION

The present application is a continuation-in-part of my copending U.S. Patent Application Serial No. 08/157,235, filed on November 24, 1993, now U.S. Patent No.                    , which is incorporated by reference in its entirety.

## Background and Introduction

The present invention concerns methods of detecting genomic RNA of hepatitis C virus (hereinafter "HCV"), methods of determining HCV genotypes, genetic nucleotides of HCV, specific primers and probes, and test kits for determining HCV genotype or for detecting HCV.

Four kinds of viral hepatitis were known as hepatitis A, B, D and E, and the presence of another hepatitis virus transmitted through blood transfusion was predicted. Post-transfusion hepatitis was remarkably reduced in Japan after introduction of diagnostic systems for screening hepatitis B virus in transfusion blood. However, there were still an estimated 280,000 annual cases of post-transfusion hepatitis presumed to be caused by another type of virus.

In 1988, Chiron Corp. claimed that it had succeeded in cloning RNA virus genome, which it termed hepatitis C virus (HCV), as the causative agent of hepatitis which was not classified in any conventional type of viral hepatitis, and reported on its partial nucleotide sequence (European Patent Application 0,318,216). HCV (c100-3) antibody detection systems based on the sequence are now being introduced for screening of transfusion bloods and for diagnosis of hepatitis in patients.

The detection systems for the c100-3 antibody have proven their partial association with HCV; however, they capture only about 70% of carriers and chronic hepatitis patients, or they fail to detect the antibody in acute phase infection, thus leaving problems yet to be solved even after development of the c100-3 antibody by Chiron Corp.

Up to today, partial or full nucleotide sequences of HCV isolates were cloned and disclosed by researchers in some laboratories and who studied the characteristics of the nucleotide sequences. As a result, many variances were found between those new nucleotide sequences and Chiron's sequence. Thus, it was elucidated that the nucleotide sequence of a sole strain could not predict the character of HCV.

Those facts suggest that the establishment of diagnosis and treatment of hepatitis requires the study and comparison of the nucleotide sequences of many HCV strains in order to reveal the genetic variation and to characterize the genomes.

The development of new diagnostic agents for screening of transfusion bloods requires studying and utilizing antigens based on a conservative region, and some specific antigens were reported including those by the inventor. In addition, raising the ability of detection in the genetic test requires utilizing the region common to strains of the virus. The inventor has already developed and established a new detection system using conservative regions which were selected from 5' non-coding region, and the system has been put to practical use.

The inventor also has found that HCV strains are classified into four major types (genotypes) regarding homology of nucleotide sequences and the presence of the type specific nucleotide sequences in a putative core region. In addition, the inventor has found a simple system of determining four genotypes by gene amplification using oligonucleotide primers having common sequences and those having type-specific sequences selected from the core region (EP 0,532,258). From the result of studies on the nucleotide sequences of HCV strains, which were isolated from bloods of hepatitis C patients collected world-wide, there was determined the presence of a new strain classified into a genotype other than the four previously disclosed genotypes.

## Summary of the Invention

One object of the present invention is to provide new oligonucleotides having the sequence specific to a new HCV strain, called type V, which could not be classified into the prevailing four genotypes, and to provide methods of detecting genomic RNA of HCV and methods of determining HCV genotypes.

The invention provides oligonucleotides shown in SEQ ID NOS:1-3, oligonucleotides having some substitutions of those nucleotides, oligonucleotides having partial sequences of those nucleotides, or oligonucleotides having complementary sequences of those nucleotides.

The invention provides methods of detecting HCV-RNA and determining the genotype of a HCV strain using the oligonucleotides described herein. The method of the invention could be carried out, for example, by using oligonucleotides shown in SEQ ID NOS:4-10, amplifying cDNAs by PCR method, and observing the

2

products or measuring the molecular size (weight or length) of the products.

In amplification of cDNAs, oligonucleotides #104 and/or #104V could be used as sense primers and one or more oligonucleotides selected from #296, #133, #134, #135 and #339 as antisense primers. When products of a prior amplification stage using oligonucleotides #256 and/or #256V as sense primers and #186 as an antisense primer were provided to the amplification stage using primers #104 and/or #104V as sense primers and one or more oligonucleotides selected from #296, #133, #134, #135 and #339 as antisense primers, more accurate results would be efficiently obtained.

A mixture of the oligonucleotides consisting of two or more of oligonucleotides #104, #104V, #296, #133, #134, #135 and #339 would be used in detection of HCV-RNA or in determination of genotype in one performance of amplification.

A kit for detection of HCV-RNA or determination of genotypes contains one or more of oligonucleotides of the invention.

The inventor examined nucleotide sequences near type specific regions used as primers in determination system, and found an oligonucleotide shown in SEQ ID NO:1. Thus a method to amplify the region common in genotypes I-V including type specific region was completed.

The inventor developed a determination system of genotypes from the combination of inventions described above; first amplification of the region common in all types is followed by second amplification of the regions inside the first amplification using type specific primers, detection of HCV-RNA, and determination of genotypes from the measurement of molecular size (weight or length) of the products.

The inventor developed a method to amplify type-specific regions, wherein the mixture of type-specific primers is used so as to work in a reaction, and to determine genotypes in the same manner irrespective of types.

The determination system of genotypes of the invention was carried out for HCV strains of types I-V. Strains of types I-IV were determined according to their types classified by the prevailing system, while strains of type V different from those types were efficiently determined from the observation of amplification products by electrophoresis or HPLC or by other known methods of comparing amplification products.

The determination system of the invention is very useful in tracing infectious sources in vertical or horizontal transmission of hepatitis C because it makes classification of HCV strains into five genotypes easy and reliable and also makes it easy to catch type-specific symptoms of hepatitis C.

A kit to determine genotypes of HCV of the invention essentially consists of one or more of oligonucleotides shown in SEQ ID NOS:1-3, and additionally consists of one or more of oligonucleotides shown in SEQ ID NOS:4-10, and optionally polymerase, molecular markers, dNTP and/or distilled water.

The method of the invention enables an easy detection of HCV-RNA, and enables also an easy, rapid and sure determination of HCV genotypes. Detection does not require that the PCR product be compared to strains of types I-V; any known method of detecting the PCR product can be utilized. The results obtained in the method is useful in prediction and estimation of the effect of treatment of patients with hepatitis C.

Oligonucleotides of the invention are useful as sense primer or antisense primer in the method of the invention.

The present invention thus concerns, but is not limited to, the following:

A recombinant oligonucleotide selected from (a) #256V having the nucleotide sequence of SEQ ID NO:1; (b) #104V having the nucleotide sequence of SEQ ID NO:2; or (c) #339 having the nucleotide sequence of SEQ ID NO:3.

A method of determining the HCV genotype of a HCV strain which involves subjecting the HCV strain to two stage PCR, wherein the first stage PCR involves utilizing antisense primer #186 (SEQ ID NO:5) and at least one of sense primers #256 (SEQ ID NO:4) and #256V (SEQ ID NO:1) for first stage amplification and wherein the second stage PCR involves utilizing at least one of sense primers #104 (SEQ ID NO:6) and #104V (SEQ ID NO:2) and at least one of antisense primers #296 (SEQ ID NO:7), #133 (SEQ ID NO:8), #134 (SEQ ID NO:9), #135 (SEQ ID NO:10), and #339 (SEQ ID NO:3) for the second stage amplification; the method further involves comparing the product of the second stage PCR with HCV genotypes I, II, III, IV, and V. The method may further involve synthesizing cDNA from viral RNA of the HCV strain and/or measuring the molecular weight of the product of the second stage PCR.

A method of determining the HCV genotype of a HCV strain which involves subjecting the HCV strain to PCR, wherein the PCR involves utilizing at least one of sense primers #104 (SEQ ID NO:6) and #104V (SEQ ID NO:2) and at least one of antisense primers #296 (SEQ ID NO:7), #133 (SEQ ID NO:8), #134 (SEQ ID NO:9), #135 (SEQ ID NO:10), and #339 (SEQ ID NO:3); the method further involves comparing the product of the PCR with HCV genotypes I, II, III, IV, and V. The method may further involve synthesizing cDNA from viral RNA of the HCV strain and/or measuring the molecular weight of the product of the second stage PCR.

A method for detecting HCV in a sample which involves subjecting the sample to two stage PCR, wherein the first stage PCR involves utilizing antisense primer #186 (SEQ ID NO:5) and at least one of sense primers #256 (SEQ ID NO:4) and #256V (SEQ ID NO:1) for first stage amplification and wherein the second stage PCR comprises utilizing at least one of sense primers #104 (SEQ ID NO:6) and #104V (SEQ ID NO:2) and at least one of antisense primers #296 (SEQ ID NO:7), #133 (SEQ ID NO:8), #134 (SEQ ID NO:9), #135 (SEQ ID NO:10), and #339 (SEQ ID NO:3) for the second stage amplification. The method may further involve synthesizing cDNA from viral RNA of the HCV and/or obtaining a sample from a patient.

A method for detecting HCV in a sample which involves subjecting the sample to PCR, wherein the PCR involves utilizing at least one of sense primers #104 (SEQ ID NO:6) and #104V (SEQ ID NO:2) and at least one of antisense primers #296 (SEQ ID NO:7), #133 (SEQ ID NO:8), #134 (SEQ ID NO:9), #135 (SEQ ID NO:10), and #339 (SEQ ID NO:3). The method may further involve synthesizing cDNA from viral RNA of the HCV and/or obtaining a sample from a patient.

A test kit for determining HCV genotype or for detecting HCV which contains at least one of SEQ ID NOS:1-3 and at least one of SEQ ID NOS:4-10. The test kit may further contain at least one of (a) dATP, dTTP, dGTP, and dCTP; (b) heat stable DNA polymerase; and (c) molecular markers.

## Brief Description of the Drawings

The present invention will be further understood with reference to the drawings, wherein:

Figure 1 is a diagram which shows the four regions in 5' terminal and 3' terminal genome of type V strain determined in Example 1.

Figure 2 shows the comparison of nucleotide sequences between (+) strand sequences corresponding to primers #256 and #186, useful in first stage amplification, and type V isolates.

Figure 3 shows the comparison of nucleotide sequences between (+) strand sequences corresponding to primers #104, #296, #133, #134 and #135, useful in second stage amplification, and type V isolates.

Figure 4 shows the comparison of nucleotide sequences between (+) strand sequences corresponding to primer #339, specific to type V strains, and isolates of genotypes I to V.

Figure 5 is a photo which shows electrophoresis pattern of genotypes I to V. Indicated on the left lane are migration positions of molecular size markers (100bp-ladder, Gibco, BRL). Lanes 1-8 show respectively HC-J1 (genotype I), HC-J4/83 (genotype II), HC-J6 (genotype III), HC-J8 (genotype IV), NZL 1 (genotype V), Th 85 (genotype V), US 114 (genotype V), and HEM 26 (genotype V).

## Detailed Description of the Invention

Determination of HCV genotypes could be useful in the following diagnostic, treatment and prevention applications;

(1) identification of infectious sources in vertical (mother-to-baby) transmission and horizontal transmission;

(2) clarification of genotype-specific features in the course, condition and prediction of recuperation of hepatitis C liver diseases and making a guide of therapy therefor;

(3) effective prevention and treatment by selecting appropriate drugs and predicting a result from sensitivity to the medicines.

The determination of genotypes is essential to obtain such applications.

The inventor discovered new strains of HCV which tested positive for HCV-RNA by the amplification of the 5' non-coding region but whose nucleotide sequences could not be classified into the four prevailing genotypes, I, II, III or IV. Thus, the strains were presumed to be classified into a new type.

The inventor sought for HCV isolates which could not be classified into the four prevailing genotypes. First, serum samples were collected from patients testing positive for HCV-RNA but which could not be classified by the prevailing method of determining genotypes. Then HCV-RNA was isolated from each serum sample and the nucleotide sequences of cDNAs were determined regarding a partial region of 5' non-coding, whole regions of core and envelope, a partial region of NS1, and a partial region of 3' terminus including 3' non-coding region.

Nucleotide sequences were compared and examined for homology concerning the new strains. As a result, it became clear that the homology among the nucleotide sequences of the new strains was more than 95%, and that those strains should be classified into a genotype. The nucleotide sequences of the four strains which were assumed to be classified into the new type were compared with the sequences of strains of types I-IV. It became clear that the four new strains had homologies as low as 69-74% with strains of types I-IV. Those new strains were confirmed to be classified into the new type named genotype V.

HCV genome is known to have some regions according to the information contained in the nucleotide sequence. Concerning type V strains, homology was calculated in each region with the strains of types I-IV. As a result, high homology (89-94%) was observed on 5' non-coding region and this region was confirmed to be conservative in all types. However, the homology was as low as 35-65% for 3' non-coding region.

From the results obtained above, the inventor found the specific homologous region among HCV strains of type V and appropriate oligonucleotides used as primers. Those primers could be used in Polymerase Chain Reaction (hereinafter called "PCR") method in order to amplify type V strains specifically, and to determine the type from the presence of amplified products.

The nucleotides of the present invention can be obtained by chemical synthesis and used for the detection of HCV by other than PCR. For instance, the nucleotides can be used as probes, and the probes work more effectively when used after being labeled with enzyme, radioisotope, luminescent substance, and other substances known in the art.

## Examples

Examples of applications of the invention are shown below; however, the invention shall in no way be limited to those examples.

Example 1-

The oligonucleotide useful for the amplification of genotype V gene was determined in the following way:

(1) Isolation of RNA

RNA from human serum or plasma samples testing positive by RNA detecting system using 5' non-coding region (EP 0,510,952), which could not be classified into any of four genotypes by the determining system using core region of HCV genome (EP 0,532,258), were isolated in the following method:

Each sample was added to the same volume of Tris chloride buffer (50mM, pH 8.0, containing 150mM NaCl, 10mM EDTA) and centrifuged at $90 \times 10^3$ rpm for 15 minutes. The precipitate was suspended in Tris chloride buffer (50mM, pH 8.0, containing 200mM NaCl, 10mM EDTA, 2% (w/v) sodium dodecyl sulfate (SDS) and proteinase K lmg/ml), and incubated at 60°C for 1 hour. Then the nucleic acids were extracted by phenol/chloroform and precipitated by ethanol to obtain RNAs.

(2) Detection of 5' non-coding region RNA by Polymerase Chain Reaction (PCR)

The isolated RNA were heated at 70°C for 1 minute; cDNA was synthesized using as a template oligonucleotide primer #299 (5'-ACCCAACACTACTCGGCTAG-3': antisense) and reverse transcriptase (Superscript, BRL, U.S.A.), and was provided to PCR as follows.

cDNA was amplified according to Saiki's method (Science (1988), volume 239, pages 487-491) using Amplitaq (Perkin-Elmer Cetus) on a DNA Thermal Cycler (Perkin-Elmer Cetus). PCR was performed in two stages using primer #32A (5'-CTGTGAGGAACTACTGTCTT-3': sense) and #299 in the first stage; and #33 (5'-TTCACGCAGAAAGCGTCTAG-3': sense) and #48 (5'-GTTGATCCAAGAAAGGACCC-3': antisense) locating inside of #32A and #299, in the second stage.

In the first stage PCR, reaction cycle was repeated 35 times with denaturation at 94°C for 1 minute, annealing of primers at 55°C for 1.5 minutes, and extension at 72°C for 3 minutes. 5ml of the products was subjected to second stage PCR for 30 cycles with denaturation at 94°C for 1 minute, annealing of primers at 55°C for 1.5 minutes, and extension at 72°C for 2 minutes.

(3) Determination of genotypes by the prevailing method

The samples confirmed positive for HCV-RNA in Example 1-(2) were subjected to the determination of genotypes by the following method:

RNAs were extracted from the samples using the method described in Example 1-(1), and cDNAs were synthesized using the method described in 1-(2), where primer #186 (5'-ATGTACCCCATGAGGTCGGC-3': antisense, SEQ ID NO:5) was used in cDNA synthesis.

The region in common with the four genotypes was amplified in the first PCR using universal primers #256 (5'-CGCGCGACGAGGAAGACTTC-3': sense, SEQ ID NO:4) and #186. The reaction cycle was repeated 35 times with denaturation at 94°C for 1 minute, annealing of primers at 55°C for 1.5 minutes, and extension at 72°C for 2 minutes.

A 1/50 amount of the products was subjected to second PCR specific for genotypes for 35 cycles using a universal primer #104 (5'-AGGAAGACTTCCGAGCGGTC-3': sense, SEQ ID NO:6) and a mixture of equivalent amount of four type-specific primers, #296 (5'-GGATAGGCTGACGTCTACCT-3': type I specific, SEQ ID NO:7), #133 (5'-GAGCCATCCTGCCCACCCCA-3': type II specific, SEQ ID NO:8), #134 (5'-CCAAGAGGGACGGGAACCTC-3': type III specific, SEQ ID NO:9) and #135 (5'-ACCCTCGTTTCCGTACAGAG-3': type IV specific, SEQ ID NO:10), with denaturation at 94°C for 1 minute, annealing of primers at 60°C for 1 minute, and extension at 72°C for 1.5 minutes. The products of the second PCR were subjected to electrophoresis on a composite agarose gel made from 1.5% NuSieve and 1.5% SeaKem (FMC BioProducts, U.S.A.), stained with ethidium bromide and observed under u.v. light. The products were identified by molecular sizes according to amplified regions and genotypes determined.

(4) Determination of nucleotide sequences of 5' terminal region and 3' terminal region

The nucleotide sequences of 5' terminal region and 3' terminal were determined on four HCV strains, NZL 1, Th 85, US 114 and HEM 26, which were positive for HCV-RNA but whose genotype could not be determined by the prevailing method, in order to examine homologies to the major genotypes.

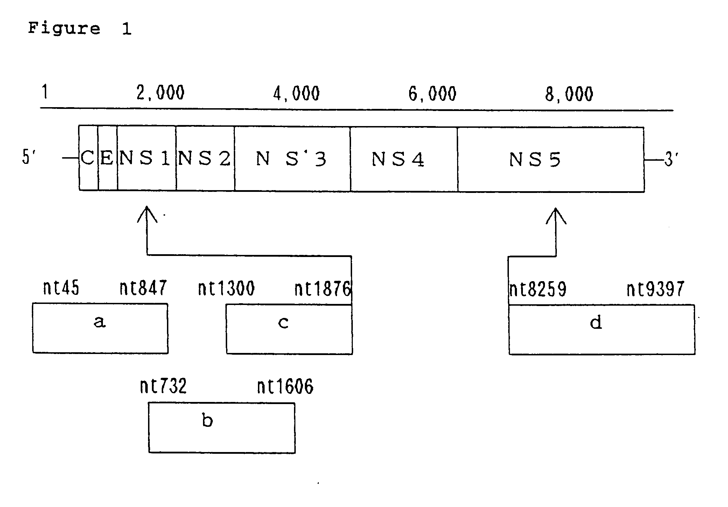

As shown in Figure 1, nucleotide sequences in four regions (a), (b), (c) and (d) were determined using specific primers by cDNA synthesis, PCR amplification and second amplification if needed. Regions (a) and (b) were designed to overlap partially each other, and (c) was also designed to overlap with (b) in the opposite site of (a), in order to cover the consecutive sequence of nt 45 to nt 1876 of 5' terminus. Primers utilized in the determination are shown in Table 1: NS5 specific primer #80 (5'-GACACCCGCTGTTTTGACTC-3'; nt 8256-8275); primer #122 (5'-AGGTTCCCTGTTGCATAGTT-3', nt 828-347) specific to the structural protein region; #50 (5'-GCCGACCTCATGGGGTACAT-3'); #165 (5'-AAGGATCCGTCGACATCGATAATACG (A) -3'); #166 (5'-AAGGATCCGTCGACATCGAT-3'); #199 (5'-GGGGTGAAACAATACACCGG-3'); #205 (5'-GGGACATGATGATCAACTGG-3');#32a (5'-CTGTGAGGAACTACTGTCTT-3'); #129 (5'-CGCATGGCATGGGACATGAT-3'); #197 (5'-GGAGTGAAGCAATACACTGG-3'); #206 (5'-GTGAAGGAATTCACTGGGCCACA-3'); #207 (5'-GTGAAAGAATTCACCGGGCCGCA-3').

## Table 1
Primers utilized in the determination of nucleotide sequence of each region

| region | cDNA primer | 1st primer | 2nd primer & literature |
|---|---|---|---|
| a (nt45–847) | #122 | #32$^\alpha$/#122 | none; Okamoto et al., Jpn J Exp Med |
| b (nt732–1606) | #337 | #50/#337 | none; Okamoto et al., J gen Virol |
| c (nt1300–1876) | #197, #199 | #129/#197, #199 | #205/#206, 207 Okamoto et al. Hept. |
| d (nt8259–9397) | #165 | #80/#166 | none; Okamoto et al., Virology |

#50 : 5'-GCCGACCTCATGGGGTACAT-3'
#337 : 5'-ATGTGCCACGAGCCATTGGT-3'

Nt numbers correspond to sequence of HC-J1 isolate.

The products of amplification were cloned into M13mp19 phage vector after treatment by T4 polynucleotide kinase and T4 DNA polymerase (Takara, Japan). Nucleotide sequences were determined by the dideoxy chain termination method using sequenase sequencing kit ver 2.0 (United States Biochemicals, U.S.A.) or AutoRead Sequencing kit (Phannacia LKB, Japan).

Table 2 shows the homologies in percentage among four strains regarding each region.

## Table 2

Homologies of nucleotide sequence among type V HCV strains (%)

| | Total region | 5'-NC | C | E | E2/NS1 | NS5 | 3'-NC |
|---|---|---|---|---|---|---|---|
| | 2905nt (855aa) | 277 | 573 (191aa) | 576 (192aa) | 360 (120aa) | 1099 (352aa) | 20 |
| NZL 1 vs | | | | | | | |
| Th 85 | 95 (95) | 99 | 98 (100) | 94 (96) | 86 (80) | 96 (98) | 95 |
| US 1 1 4 | 95 (96) | 99 | 98 (100) | 95 (98) | 86 (78) | 96 (98) | 95 |
| HEM 2 6 | 96 (96) | 99 | 98 (99) | 96 (97) | 88 (81) | 97 (99) | 95 |
| Th 8 5 vs | | | | | | | |
| US 1 1 4 | 96 (96) | 99 | 98 (100) | 96 (95) | 87 (81) | 97 (99) | 100 |
| HEM 2 6 | 95 (95) | 99 | 98 (99) | 94 (94) | 86 (80) | 96 (98) | 100 |
| US 1 1 4 vs | | | | | | | |
| HEM 2 6 | 95 (95) | 99 | 98 (99) | 94 (94) | 86 (80) | 96 (98) | 100 |

As a result, though some difference in percentage were observe in each region, the homologies were observed to be very high (more than 95%) for every two strains of the four in comparison to the whole region.

Table 3 shows the comparison of nucleotide sequences between the four strains (new strains NZL 1, Th 85, US 114 and HEM 26) which are determined in the Example and four strains (prevailing strains HC-J1, HC-J4, HC-J6, HC-J8) whose genotypes were identified by the prevailing method.

Table 3

Homologies of nucleotide sequence between type V HCV strains and types I-IV (%)

| | Total region | 5'-NC | C | E | E2/NS1 | NS5 | 3'-NC |
|---|---|---|---|---|---|---|---|
| | 2905nt (855aa) | 277 | 573 (191aa) | 576 (192aa) | 360 (120aa) | 1099 (352aa) | 20 |
| **NZL 1 vs** | | | | | | | |
| HC-J 1 (I) | 73(73) | 94 | 84(91) | 65(66) | 65(59) | 71(74) | 45 |
| HC-J 4 (II) | 73(75) | 94 | 82(91) | 65(69) | 62(60) | 71(75) | 60 |
| HC-J 6 (III) | 70(71) | 91 | 82(89) | 60(56) | 62(53) | 68(75) | 35 |
| HC-J 8 (IV) | 69(72) | 89 | 81(88) | 58(56) | 59(57) | 68(76) | 40 |
| **Th 8 5 vs** | | | | | | | |
| I | 74(74) | 93 | 84(91) | 63(67) | 68(60) | 71(75) | 50 |
| II | 73(75) | 94 | 82(91) | 65(69) | 61(60) | 71(76) | 65 |
| III | 70(71) | 91 | 82(89) | 59(56) | 60(54) | 68(75) | 35 |
| IV | 69(72) | 90 | 81(88) | 58(57) | 57(57) | 69(77) | 35 |
| **US 1 1 4 vs** | | | | | | | |
| I | 73(73) | 94 | 84(91) | 64(66) | 63(54) | 71(75) | 50 |
| II | 73(74) | 94 | 82(91) | 66(69) | 59(51) | 71(76) | 65 |
| III | 70(71) | 91 | 82(89) | 59(55) | 63(53) | 68(76) | 35 |
| IV | 69(72) | 90 | 81(89) | 57(55) | 58(53) | 69(77) | 35 |
| **HEM2 6 vs** | | | | | | | |
| I | 74(74) | 94 | 84(90) | 65(66) | 64(59) | 72(74) | 50 |
| II | 73(74) | 94 | 82(90) | 66(68) | 60(58) | 71(75) | 65 |
| III | 70(71) | 91 | 81(87) | 59(55) | 61(55) | 69(76) | 35 |
| IV | 69(72) | 90 | 81(88) | 58(56) | 57(53) | 69(77) | 35 |

As a result, homologies less than 95% were observed in 5' non-coding region, in which nucleotide sequences are considered most conservative, between new strains and prevailing strains. The homologies were obviously lower than those among new strains (ca. 99%). The homologies in the other regions were observed to be 35% to 84%, lower than those in 5' non-coding region, and were significantly lower than those among the four new strains in any region.

The homologies in each region were calculated as follows;

[among four new strains]-[between new strain and prevailing strain]

core region;        [98%] - [81-84%]

envelope region;      [94-96%] - [57-66%]
E2/NS1 region;     [85-88%] - [57-68%]
NS5 region;       [96-97%] - [68-72%]
3' non-coding region;    [95-100%] - [35-65%]

The results show that the four new strains, positive for HCV-RNA but impossible to be type-classified according to the prevailing system, should be classified into a single and new type which does not belong to types I-IV. The new type was called type V because the strains had high homologies to putative type V or group IV in specific region.

## (5) Determination of type-V-specific primers

The region having the characteristics described below was examined from the nucleotide sequences of the new strains;

(a) having common sequence with type V strains,

(b) having low homologies with the other types, and would not amplify the others,

(c) locating inside the region used in the prevailing determination system of genotypes.

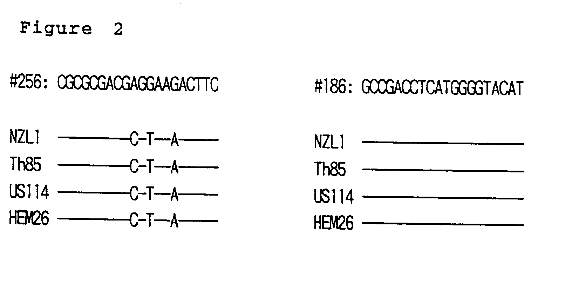

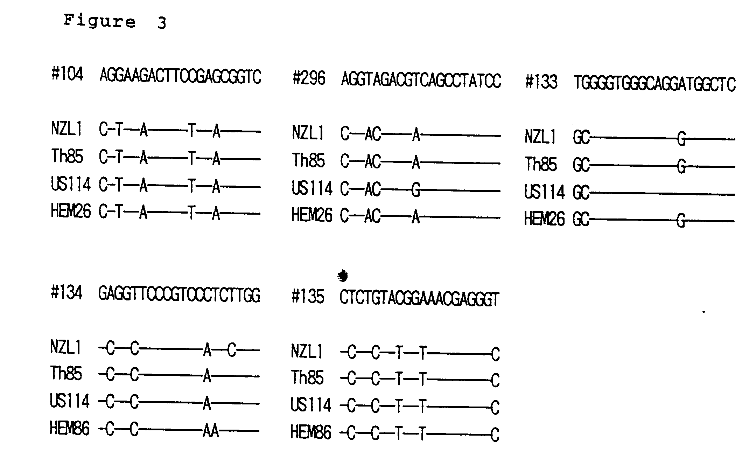

Concerning the type V strains, the nucleotide sequences corresponding to those of primers used in the prevailing system were compared and examined (see Figure 2 & Figure 3). Regarding universal primer #256, some mismatches were observed in this region, thus it was not considered appropriate for the amplification of type V; regarding universal #186, it is possible to be used because homologous sequence was observed in the whole region.

Regarding primers used in the second stage (type-specific) amplification: (1) universal sense primer #104, many mismatches were observed, thus it was not considered appropriate; but (2) type-specific antisense primers #296, #133, #134 and #135 having 3 to 5 mismatches were considered possible to be used because those primers could not amplify type V.

In order to raise affinity to type V nucleotide sequences, primer #256V (SEQ ID NO:1) which sequence was changed in three nucleotides, which would be used as a sense primer in the first stage (universal) amplification instead of #256, was determined.

Then #104V (SEQ ID NO:2) was determined by the modification of #104 which would be used as a sense primer in the second stage (type-specific) amplification instead of #104.

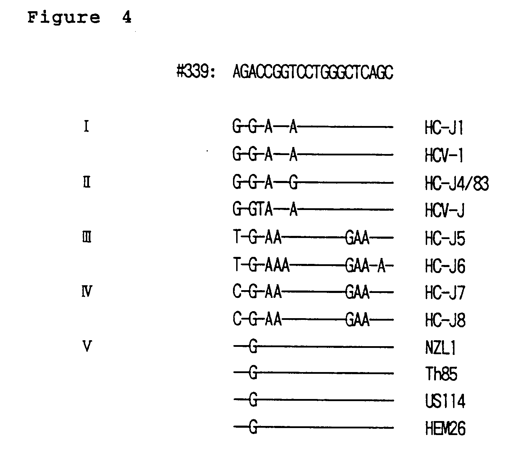

Primer #339 (SEQ ID NO:3) was also determined as type V-specific antisense primer used in the second stage amplification. Regarding primer #339, 4-9 mismatches were observed when compared to strains of types I-IV (see Figure 4); thus, this primer could not amplify types I-IV.

## (6) Synthesis of oligonucleotides

Said oligonucleotides #256V, #104V, #339, #256, #186, #104, #296, #133, #134 and #135 were synthesized with cyclone Plus DNA Synthesizer (MilliGen/Biosearch, Division of Millipore) in the usual way.

## Example 2-

HCV genotypes were determined using primers #256V, #104V and #339 in the following way:

HCV genotypes were determined using oligonucleotides obtained in Example 1-(6) as primers. HCV strains whose genotypes were already determined were employed for each type: HC-J1 as type I, HC-J4/83 as type II, HC-J6 as type III, HC-J8 as type IV, and NZL 1, Th 85, US 114 and HEM 26 were used.

cDNAs were synthesized in the usual way using #186 as a primer, followed by first stage (universal) amplification using #256 and #256V as sense primers, and #186 as an antisense primer. The reaction cycle was repeated 35 times with denaturation at 94°C for 1 minute, annealing of primers at 55°C for 1 minute, and extension at 72°C for 1.5 minutes, followed by extension at 72°C for 7 minutes. The products were subjected to the second (type-specific) amplification using #104 and #104V as sense primers and #296, #133, #134, #135 and #339 as antisense primers, where the primers were used in a mixture of equivalent amounts. The reaction cycle was repeated 35 times with denaturation at 94°C for 30 seconds, annealing of primers at 60°C for 30 seconds, and extension at 72°C for 30 seconds, followed by extension at 72°C for 7 minutes.

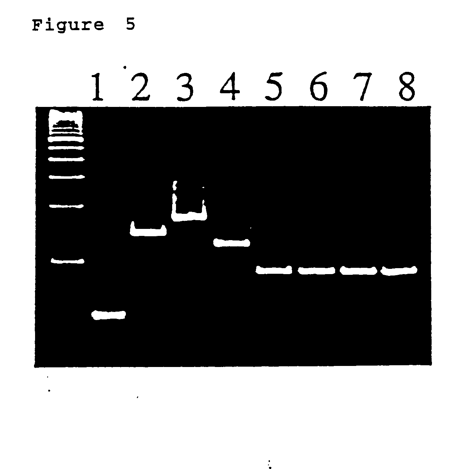

The products were subjected to electrophoresis on a composite agarose gel made from 2% NuSieve and 2% SeaKem (FMC BioProducts, U.S.A.), stained with ethidium bromide and observed under u.v. light (see Figure 5). As a result, strain types I-IV were identified as predicted, whereas new strains of type V showed products having a molecular size (88bp) which was predicted from designed primer pair. Thus, it was confirmed that the position of the product observed would show that the product was obtained from type V strain. In addition, because no interference was observed in any strains, it was confirmed that false reactions did not occur and that the method of the present invention was very useful in view of accuracy.

Thus, the present invention provides a method to detect HCV with high fidelity, and primers and probes to use in the method to detect HCV. Also the invention provides polynucleotides and oligonucleotides which could give important information to design primers and probes to detect HCV.

It will also be understood that the practice of the present invention is not limited to the use of the exact sequence of SEQ ID NOS:1-10. Modifications to the sequence, such as deletions, insertions, or substitutions in the sequence are also contemplated. For example, alteration in the gene sequence which reflect the degeneracy of the genetic code are contemplated. Therefore, where the phrase SEQ ID NOS:1-10 is used in either the specification or the claims, it will be understood to encompass all such modifications and variations. In particular, the invention contemplates those nucleic acid sequences which are sufficiently duplicative of SEQ ID NOS:1-10 so as to permit hybridization therewith under standard high stringency southern hybridization conditions, such as those described in Maniatis et al. (Molecular Cloning. A Laboratory Manual, 2nd ed. Cold Spring Harbor Laboratory, 1989).

Further variations and modifications of the foregoing will be apparent to those skilled in the art and such variations and modifications are attended to be encompassed by the claims that are appended hereto.

U.S. Patent Application Serial No. 07/940,242, filed on September 8, 1992, now U.S. Patent No. , is incorporated by reference in its entirety.

Japanese Priority Application 179753/93, filed on June 14, 1993, is relied on and incorporated by reference.

Various primers and techniques utilized in the present application are also described in the following published European Patent Applications: EP 0,441,664; EP 0,461,863; EP 0,485,209; EP 0,488,812; EP 0,500,236; EP 0,516,270; EP 0,510,952; EP 0,532,167; EP 0,532,258; and EP 0,554,624.

SEQ ID NO:1 (primer #256V)
(A) Length: 20 base pairs
(B) Type: nucleic acid
(C) Strandedness: single
(D) Topology: linear
(E) Molecular type:  cDNA to genomic RNA

Sequence Description of SEQ ID NO:1
CGCGCGACGC GTAAAACTTC 20


SEQ ID NO:2 (primer #104V)
(A) Length: 20 base pairs
(B) Type: nucleic acid
(C) Strandedness: single
(D) Topology: linear
(E) Molecular type: cDNA to genomic RNA

Sequence Description of SEQ ID NO:2
CGTAAAACTT CTGAACGGTC 20


SEQ ID NO:3 (primer #339)
(A) Length: 20 base pairs
(B) Type: nucleic acid
(C) Strandedness: single
(D) Topology: linear
(E) Molecular type: cDNA to genomic RNA

Sequence Description of SEQ ID NO:3
GCTGAGCCCA GGACCGGTCT 20


SEQ ID NO:4 (primer #256)
(A) Length: 20 base pairs
(B) Type: nucleic acid
(C) Strandedness: single
(D) Topology: linear
(E) Molecular type: cDNA to genomic RNA

Sequence Description of SEQ ID NO:4
CGCGCGMCNA GGAARRCTTC 20
(M=A/C; N=A/T/C/G; R=A/G)

SEQ ID NO:5 (primer #186)
(A) Length: 20 base pairs
(B) Type: nucleic acid
(C) Strandedness: single
(D) Topology: linear
(E) Molecular type: cDNA to genomic RNA

Sequence Description of SEQ ID NO:5
AYGTACCCCA YGAGRTCGGC 20
(Y=T/C: R=G/A)

SEQ ID NO:6 (primer #104)
(A) Length: 20 base pairs
(B) Type: nucleic acid
(C) Strandedness: single
(D) Topology: linear
(E) Molecular type: cDNA to genomic RNA

Sequence Description of SEQ ID NO:6
AGRAARRCTT CSGAGCGRTC 20
(R=G/A; S=C/G)

SEQ ID NO:7 (primer #296)
(A) Length: 20 base pairs
(B) Type: nucleic acid
(C) Strandedness: single
(D) Topology: linear
(E) Molecular type: cDNA to genomic RNA

Sequence Description of SEQ ID NO:7
GGATAGGCTG ACGTCTACCT 20

SEQ ID NO:8 (primer #133)
(A) Length: 20 base pairs
(B) Type: nucleic acid
(C) Strandedness: single
(D) Topology: linear
(E) Molecular type: cDNA to genomic RNA

Sequence Description of SEQ ID NO:8
GARCCAWCCT GCCCAYCCYA 20
(R=G/A; W=T/A; Y=C/T)

```
SEQ ID NO:9 (primer #134)
(A) Length: 20 base pairs
(B) Type: nucleic acid
(C) Strandedness: single
(D) Topology: linear
(E) Molecular type: cDNA to genomic RNA

Sequence Description of SEQ ID NO:9
CCAARAGGGA CGGGARCCTC 20
(R=G/A)
```

```
SEQ ID NO:10 (primer #135)
(A) Length; 20 base pairs
(B) Type nucleic acid
(C) Strandedness: single
(D) Topology: linear
(E)Molecular type: cDNA to genomic RNA

Sequence Description of SEQ ID NO:10
RCCYTCGTTT CCRTACAGRG 20
(R=G/A; Y=C/T)
```

## Claims

1. A recombinant oligonucleotide selected from the group consisting of:
   (a) #256V having the nucleotide sequence of SEQ ID NO:1;
   (b) #104V having the nucleotide sequence of SEQ ID NO:2; or
   (c) #339 having the nucleotide sequence of SEQ ID NO:3.

2. A method of determining the HCV genotype of a HCV strain, said method comprising subjecting said HCV strain to two stage PCR, wherein the first stage PCR comprises utilizing antisense primer #186 (SEQ ID NO:5) and at least one of sense primers #256 (SEQ ID NO:4) and #256V (SEQ ID NO:1) for first stage amplification and wherein the second stage PCR comprises utilizing at least one of sense primers #104 (SEQ ID NO:6) and #104V (SEQ ID NO:2) and at least one of antisense primers #296 (SEQ ID NO:7), #133 (SEQ ID NO:8), #134 (SEQ ID NO:9), #135 (SEQ ID NO:10), and #339 (SEQ ID NO:3) for the second stage amplification; said method further comprising comparing the product of said second stage PCR with HCV genotypes I, II, III, IV, and V.

3. The method according to claim 2, further comprising synthesizing cDNA from viral RNA of said HCV strain.

4. The method according to claim 2, further comprising measuring the molecular weight of the product of said second stage PCR.

5. A method of determining the HCV genotype of a HCV strain, said method comprising subjecting said HCV strain to PCR, wherein said PCR comprises utilizing at least one of sense primers #104 (SEQ ID NO:6) and #104V (SEQ ID NO:2) and at least one of antisense primers #296 (SEQ ID NO:7), #133 (SEQ ID NO:8), #134 (SEQ ID NO:9), #135 (SEQ ID NO:10), and #339 (SEQ ID NO:3); said method further comprising comparing the product of said PCR with HCV genotypes I, II, III, IV, and V.

6. The method according to claim 5, further comprising synthesizing cDNA from viral RNA of said HCV strain.

7. The method according to claim 5, further comprising measuring the molecular weight of the product of said PCR.

8. A method for detecting HCV in a sample, said method comprising subjecting said sample to two stage PCR, wherein the first stage PCR comprises utilizing antisense primer #186 (SEQ ID NO:5) and at least one of sense primers #256 (SEQ ID NO:4) and #256V (SEQ ID NO:1) for first stage amplification and wherein the second stage PCR comprises utilizing at least one of sense primers #104 (SEQ ID NO:6) and #104V (SEQ ID NO:2) and at least one of antisense primers #296 (SEQ ID NO:7), #133 (SEQ ID NO:8), #134 (SEQ ID NO:9), #135 (SEQ ID NO:10), and #339 (SEQ ID NO:3) for the second stage amplification.

9. The method according to claim 8, further comprising synthesizing cDNA from viral RNA of said HCV.

10. The method according to claim 8, further comprising obtaining a sample from a patient.

11. A method for detecting HCV in a sample, said method comprising subjecting said sample to PCR, wherein said PCR comprises utilizing at least one of sense primers #104 (SEQ ID NO:6) and #104V (SEQ ID NO:2) and at least one of antisense primers #296 (SEQ ID NO:7), #133 (SEQ ID NO:8), #134 (SEQ ID NO:9), #135 (SEQ ID NO:10), and #339 (SEQ ID NO:3).

12. The method according to claim 11, further comprising synthesizing cDNA from viral RNA of said HCV.

13. The method according to claim 11, further comprising obtaining a sample from a patient.

14. A test kit for determining HCV genotype or for detecting HCV, said kit comprising at least one of SEQ ID NOS:1-3 and at least one of SEQ ID NOS:4-10.

15. The test kit according to claim 14, further comprising at least one of (a) dATP, dTTP, dGTP, and dCTP; (b) heat stable DNA polymerase; and (c) molecular markers.

Figure 1

## Figure 2

#256: CGCGCGACGAGGAAGACTTC

#186: GCCGACCTCATGGGGTACAT

Figure 3

#104  AGGAAGACTTCCGAGCGGTC        #296  AGGTAGACGTCAGCCTATCC        #133  TGGGGTGGGCAGGATGGCTC

NZL1  C-T—A———T—A———          NZL1  C—AC———A————          NZL1  GC——————————G———
Th85  C-T—A———T—A———          Th85  C—AC———A————          Th85  GC——————————G———
US114 C-T—A———T—A———          US114 C—AC———G————          US114 GC——————————————
HEM26 C-T—A———T—A———          HEM26 C—AC———A————          HEM26 GC——————————G———


#134  GAGGTTCCCGTCCCTCTTGG        #135  CTCTGTACGGAAACGAGGGT

NZL1  -C—C————A—C—           NZL1  -C—C—T—T————C
Th85  -C—C————A———           Th85  -C—C—T—T————C
US114 -C—C————A———           US114 -C—C—T—T————C
HEM86 -C—C————AA——           HEM86 -C—C—T—T————C

Figure 4

#339:   AGACCGGTCCTGGGCTCAGC

| I | G-G-A—A——————— | HC-J1 |
| | G-G-A—A——————— | HCV-1 |
| II | G-G-A—G——————— | HC-J4/83 |
| | G-GTA—A——————— | HCV-J |
| III | T-G-AA————GAA— | HC-J5 |
| | T-G-AAA———GAA-A- | HC-J6 |
| IV | C-G-AA————GAA— | HC-J7 |
| | C-G-AA————GAA— | HC-J8 |
| V | —G——————————— | NZL1 |
| | —G——————————— | Th85 |
| | —G——————————— | US114 |
| | —G——————————— | HEM26 |

Figure 5

EP 0 633 321 A1

European Patent Office

**EUROPEAN SEARCH REPORT**

Application Number

EP 94 30 4237

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X<br>Y | EP-A-0 532 258 (IMMUNO JAPAN INC)<br>* page 5; example 1 *<br><br>* page 6, line 15 - line 25 *<br>--- | 11-13<br>1-10,14,<br>15 | C12Q1/70<br>C12Q1/68<br>C07H21/04<br>C12P19/34 |
| Y | WO-A-92 19743 (CHIRON CORP)<br><br>* page 9, line 20 - page 10, line 3 *<br>* page 21, line 1 - line 10 *<br>* page 23, line 15 - line 27 *<br>* page 38, line 15 - line 27 *<br>* figures 4A-4C *<br>* claims 21-36,51-67 *<br>--- | 1-10,14,<br>15 | |
| Y<br><br>A | EP-A-0 461 863 (IMMUNO JAPAN INC.)<br><br>* the whole document *<br>--- | 1-10,14,<br>15<br>11-13 | |
| A | BIOCHEMICAL AND BIOPHYSICAL RESEARCH COMMUNICATIONS,<br>vol.28, no.3, February 1992, DULUTH, MN US<br>pages 334 - 342<br>MORI, S. ET AL 'a new type of hepatitis c virus in patients in Thailand'<br>* page 340; figure 2 *<br>--- | 1 | TECHNICAL FIELDS SEARCHED (Int.Cl.5)<br><br>C12Q |
| A | WO-A-92 02642 (CHIRON CORP)<br>* page 23, line 10 - page 25, line 19 *<br>----- | 1 | |

The present search report has been drawn up for all claims

| Place of search | Date of completion of the search | Examiner |
|---|---|---|
| THE HAGUE | 11 October 1994 | Osborne, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)